(19) Europäisches Patentamt
European Patent Office
Office européen des brevets

(11) EP 2 346 987 B1

(12) **EUROPEAN PATENT SPECIFICATION**

(45) Date of publication and mention
of the grant of the patent:
**17.02.2016 Bulletin 2016/07**

(21) Application number: **09783803.1**

(22) Date of filing: **07.10.2009**

(51) Int Cl.:
*C12N 1/34* $^{(2006.01)}$        *C07K 14/37* $^{(2006.01)}$

(86) International application number:
**PCT/EP2009/063006**

(87) International publication number:
**WO 2010/043520 (22.04.2010 Gazette 2010/16)**

(54) **HYDROPHOBIN SOLUTION CONTAINING ANTIFOAM**

HYDROPHOBINLÖSUNG MIT ANTISCHAUMMITTEL

SOLUTION D'HYDROPHOBINE CONTENANT DE L'ANTIMOUSSE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **16.10.2008 PCT/EP2008/063929**

(43) Date of publication of application:
**27.07.2011 Bulletin 2011/30**

(73) Proprietors:
• **Unilever PLC**
**London**
**Greater London EC4P 4BQ (GB)**
Designated Contracting States:
**CY GB IE MT**
• **Unilever N.V.**
**3013 AL Rotterdam (NL)**
Designated Contracting States:
**AT BE BG CH CZ DE DK EE ES FI FR GR HR HU IS IT LI LT LU LV MC MK NL NO PL PT RO SE SI SK SM TR**

(72) Inventors:
• **COX, Andrew, Richard**
**Bedford Bedfordshire MK44 1LQ (GB)**

• **RUSSELL, Andrew, Baxter**
**Bedford Bedfordshire MK44 1LQ (GB)**
• **TIER, Christopher, Mark**
**Bedford Bedfordshire MK44 1LQ (GB)**

(74) Representative: **van Benthum, Wilhelmus A. J.**
**Unilever N.V.**
**Unilever Patent Group**
**Olivier van Noortlaan 120**
**3133 AT Vlaardingen (NL)**

(56) References cited:
**WO-A-2009/050222**

• **BAILEY M J ET AL: "Process Technological effects of deletion and amplification of hydrophobins I and II in transformants of Trichoderma reesei" APPLIED MICROBIOLOGY AND BIOTECHNOLOGY, SPRINGER VERLAG, BERLIN, DE, vol. 58, 1 January 2002 (2002-01-01), pages 721-727, XP003015238 ISSN: 0175-7598 cited in the application**

Remarks:
The file contains technical information submitted after the application was filed and not included in this specification

## Description

### Technical Field of the Invention

The present invention relates to a hydrophobin solution containing antifoam. In particular it relates to a hydrophobin solution containing antifoam obtained through a fermentation process.

### Background to the Invention

Foaming is a common problem in aerobic, submerged fermentations. Foaming is caused by the sparging of gas into the fermentation medium for the purpose of providing oxygen for the growth of the aerobic organism being cultivated (e.g. bacteria, yeasts, fungi, algae, cell cultures). If the fermentation medium contains surface active components such as proteins, polysaccharides or fatty acids, then foam can be formed on the surface of the medium as the sparged gas bubbles disengage from the liquid. Foaming creates a number of problems including the undesirable stripping of product, nutrients, and cells into the foam, and can make process containment difficult. A known method for controlling foaming is to use antifoams, of which several types are commonly used: silicone-based (e.g. polydimethylsiloxanes), polyalkylene glycols (e.g. polypropylene glycol), fatty acids, polyesters and natural oils (e.g. linseed oil, soybean oil). Antifoams replace foam-forming components on bubble surfaces, resulting in destruction of the foam by bubble coalescence. Antifoams are added at the start of and / or during the fermentation.

When the fermentation product is intended for use in foods, personal products or medicine, it is highly desirable that the product is excreted by the producing organism into the fermentation medium (i.e. extra-cellular, rather than intra-cellular production). This avoids the need to disrupt the cells by physical or chemical means in order to release the product for recovery. By maintaining the cells intact, the cellular material can be easily separated from the product so that it is free of intracellular and genetic material which is usually regarded as an undesirable contaminant. This can be especially important when the producing organism has been genetically modified. However, extra-cellular production of a hydrophobin may intensify the degree of foaming in the fermenter. The use of antifoams presents a particular problem in the extra-cellular production of hydrophobin for two reasons: firstly the amount of antifoam required is increased because the hydrophobin itself contributes to foaming in the fermenter. Secondly, the antifoam must be substantially removed since the presence of antifoam together with the hydrophobin will impair the hydrophobin functionality.

Bailey et al, Appl. Microbiol. Biotechnol. 58 (2002) pp 721-727 disclose the production of hydrophobins HFB I and HFB II by the fermentation of transformants of *Trichoderma reesei.* An antifoam (Struktol J633) was used to prevent foaming and the hydrophobin was purified using aqueous two phase extraction.

It has now been found that a certain level of antifoam can be present in the hydrophobin solution while the hydrophobin retains at least part of its functionality. It is thus possible to have a hydrophobin solution containing antifoam, therefore simplifying its production process and leading to significant savings.

### Brief Description of the Invention

It is the object of the present invention to provide an aqueous solution containing at least 300 mg/l of hydrophobin and at least 0.3 mg/l of antifoam, wherein the antifoam/hydrophobin weight ratio is below 0.2, preferably below 0.15, more preferably below 0.1 and wherein the antifoam has a cloud point.

Preferably, the aqueous solution contains at least 0.5 mg/l of antifoam.

Preferably also, the hydrophobin is a class II hydrophobin, most preferably HFBI or HFBII from *Trichoderma reesei.*

Preferably the aqueous solution is concentrated so that the antifoam/hydrophobin ratio remains below 0.2 but its hydrophobin content is above 1 g/l, preferably 10g/l even more preferably 100g/l.

### Detailed Description of the Invention

Unless defined otherwise, all technical and scientific terms used herein have the same meaning as commonly understood by one of ordinary skill in the art (e.g. in cell culture, molecular genetics, nucleic acid chemistry, hybridisation techniques and biochemistry). Standard techniques used for molecular and biochemical methods can be found in Sambrook et al., Molecular Cloning: A Laboratory Manual, 3rd ed. (2001) Cold Spring Harbor Laboratory Press, Cold Spring Harbor, N.Y. and Ausubel et al., Short Protocols in Molecular Biology (1999) 4th Ed, John Wiley & Sons, Inc. - and the full version entitled Current Protocols in Molecular Biology.

Hydrophobins

Hydrophobins can be obtained by culturing filamentous fungi such as hyphomycetes (e.g. *Trichoderma*), basidiomycetes and ascomycetes. Particularly preferred hosts are food grade organisms, such as *Cryphonectria parasitica* which secretes a hydrophobin termed cryparin (MacCabe and Van Alfen, 1999, App. Environ. Microbiol 65: 5431-5435). Similarly, surfactin can be obtained from *Bacillus subtilis* and glycolipids from e.g. *Pseudomanas aeruginosa, Rhodococcus erythropolis, Mycobacterium* species and *Torulopsis bombicola* (Desai and Banat, Microbiology and Molecular Biology Reviews, Mar. 1997, pp 47-64).

In EP 1 623 631 we have previously found that hydrophobins allow the production of aqueous foams with excellent stability to disproportionation and coalescence. Because hydrophobins are highly effective foaming agents, their presence in the fermentation medium presents a particular challenge for foam control.

Hydrophobins are a well-defined class of proteins (Wessels, 1997, Adv. Microb. Physio. 38: 1-45; Wosten, 2001, Annu Rev. Microbiol. 55: 625-646) capable of self-assembly at a hydrophobic/hydrophilic interface, and having a conserved sequence:

$$X_n\text{-}C\text{-}X_{5\text{-}9}\text{-}C\text{-}C\text{-}X_{11\text{-}39}\text{-}C\text{-}X_{8\text{-}23}\text{-}C\text{-}X_{5\text{-}9}\text{-}C\text{-}C\text{-}X_{6\text{-}18}\text{-}C\text{-}X_m \text{ (SEQ ID No. 1)}$$

where X represents any amino acid, and n and m independently represent an integer. Typically, a hydrophobin has a length of up to 125 amino acids. The cysteine residues (C) in the conserved sequence are part of disulphide bridges. In the context of the present invention, the term hydrophobin has a wider meaning to include functionally equivalent proteins still displaying the characteristic of self-assembly at a hydrophobic-hydrophilic interface resulting in a protein film, such as proteins comprising the sequence:

$$X_n\text{-}C\text{-}X_{1\text{-}50}\text{-}C\text{-}X_{0\text{-}5}\text{-}C\text{-}X_{1\text{-}100}\text{-}C\text{-}X_{1\text{-}100}\text{-}C\text{-}X_{1\text{-}50}\text{-}C\text{-}X_{0\text{-}5}\text{-}C\text{-}X_{1\text{-}50}\text{-}C\text{-}X_m \text{ (SEQ ID No. 2)}$$

or parts thereof still displaying the characteristic of self-assembly at a hydrophobic-hydrophilic interface resulting in a protein film. In accordance with the definition of the present invention; self-assembly can be detected by adsorbing the protein to Teflon and using Circular Dichroism to establish the presence of a secondary structure (in general, $\alpha$-helix) (De Vocht et al., 1998, Biophys. J. 74: 2059-68).

The formation of a film can be established by incubating a Teflon sheet in the protein solution followed by at least three washes with water or buffer (Wosten et al., 1994, Embo. J. 13: 5848-54). The protein film can be visualised by any suitable method, such as labeling with a fluorescent marker or by the use of fluorescent antibodies, as is well established in the art. m and n typically have values ranging from 0 to 2000, but more usually m and n in total are less than 100 or 200. The definition of hydrophobin in the context of the present invention includes fusion proteins of a hydrophobin and another polypeptide as well as conjugates of hydrophobin and other molecules such as polysaccharides.

Hydrophobins identified to date are generally classed as either class I or class II. Both types have been identified in fungi as secreted proteins that self-assemble at hydrophobilic interfaces into amphipathic films. Assemblages of class I hydrophobins are generally relatively insoluble whereas those of class II hydrophobins readily dissolve in a variety of solvents. Preferably the hydrophobin is soluble in water, by which is meant that it is at least 0.1% soluble in water, preferably at least 0.5%. By at least 0.1% soluble is meant that no hydrophobin precipitates when 0.1g of hydrophobin in 99.9 mL of water is subjected to 30,000 g centrifugation for 30 minutes at 20°C.

Hydrophobin-like proteins (e.g."chaplins") have also been identified in filamentous bacteria, such as *Actinomycete* and *Streptomyces* sp. (WO01/74864; Talbot, 2003, Curr. Biol, 13: R696-R698). These bacterial proteins by contrast to fungal hydrophobins, may form only up to one disulphide bridge since they may have only two cysteine residues. Such proteins are an example of functional equivalents to hydrophobins having the consensus sequences shown in SEQ ID Nos. 1 and 2, and are within the scope of the present invention.

More than 34 genes coding for hydrophobins have been cloned, from over 16 fungal species (see for example WO96/41882 which gives the sequence of hydrophobins identified in *Agaricus bisporus;* and Wosten, 2001, Annu Rev. Microbiol. 55: 625-646). For the purpose of the invention hydrophobins possessing at least 80% identity at the amino acid level to a hydrophobin that naturally occurs are also embraced within the term "hydrophobins".

Antifoams

The term "antifoam" includes both antifoams which are usually added before foaming occurs and also those which are usually added once the foam has formed (sometimes known as defoamers). A definition of antifoams used in the present invention is found in "Foam and its mitigation in fermentation systems" - Beth Junker - Biotechnology Progress, 2007, 23, 768-784

A specific group of antifoams suitable for use in the present invention are those that exhibit a cloud point. The cloud point is the temperature at which an aqueous solution of the antifoam becomes visibly turbid as it phase separates (i.e. the antifoam molecules form aggregates which scatter light) as described on p63 of Surfactant Aggregation and Adsorption at Interfaces, J. Eastoe, in Colloid Science: Principles, Methods and Applications, ed. T. Cosgrove, Blackwell Publishing, 2005.

Examples of antifoams which display cloud points include poly(alkylene glycol) (PAG) based compounds such as ethylene oxide/propylene oxide block co-polymers, polyalcohols based on ethylene oxide/propylene oxide block copolymers and polyethers of ethylene and propylene oxides; and fatty acid ester-based compounds.

The cloud point depends on the surfactant composition and chemical structure. For example, for polyoxyethylene (PEO) non-ionic surfactants, the cloud point increases as the EO content increases for a given hydrophobic group. Preferably the cloud point of the antifoam is between 0°C and 90°C, more preferably between 5° and 60°C.

Preferably, the antifoam comprises at least one non-ionic surfactant/polymer, such as a polyether, a poly(alkylene glycol), an ethylene/propylene oxide block co-polymer, a polyalcohol based on an ethylene/propylene oxide block co-polymer, a polypropylene glycol-based polyether dispersion, or an alkoxylated fatty acid ester. PAG-based antifoams (such as Struktol J647 obtainable from Schill and Seilacher), polyalcohols based on EO/PO block co-polymers (such as Struktol J647 obtainable from Schill and Seilacher) and other non-ionic surfactant antifoams are particularly effective at destroying foam, even in the presence of powerful foaming agents such as hydrophobin.

Mixtures of antifoams can be used, in which case, the cloud point of such a mixture is defined as the highest cloud point of the individual components.

Some common commercially available antifoams that exhibit a cloud point are shown in Table 1.

Table 1

| Antifoam | Cloud Point / °C |
|---|---|
| **Poly(alkylene glycol)** | |
| Struktol J647, Schill & Seilacher | 24 |
| Struktol SB2121 | *ca.* 30 |
| UCON LB 65, Dow Chemical Company | 25 |
| UCON LB 285 | 15 |
| UCON LB 625 | 10 |
| UCON LB 1715 | 8 |
| KFO673, Lubrizol | 25 |
| ST934, Pennwhite Ltd | ca. 20 |
| **Ethylene/propylene oxide block co polymers** | |
| Pluronic PE3100, BASF | 41 |
| Pluronic PE6100 | 23 |
| Pluronic PE6200 | 33 |
| Pluronic PE8100 | 36 |
| Pluronic PE10100 | 35 |
| Mazu DF204, BASF | 18-21 |
| **Polyalcohol based on EO/PO block co polymer** | |
| Struktol J650, Schill & Seilacher | 13 |
| **Polypropylene glycol based polyether dispersions** | |
| Antifoam 204, Sigma | 15 |
| **Alkoxylated fatty acid ester** | |
| Struktol J673, Schill & Seilacher | 30 |

Antifoam Measurement Method

The concentrations of the antifoam in the filtrates were determined by using the Lange LCK 433 Water Testing Kit for non-ionic surfactants. This uses the principle that non-ionic surfactants (such as J647) form complexes with the indicator TBPE (tetrabromophenolphthalein ethyl ester), which can be extracted in dichloromethane and photometrically measured to determine the concentration. First, a calibration curve was constructed. A 0.3% (w/v) solution of Struktol J647 was prepared by taking an aliquot of 3.00 g Struktol J647 and diluting to 1 L with MilliQ water at 15°C. Aliquots were taken from this and diluted with MilliQ water to give concentrations of: 6, 15, 30, 60, 150 and 300 mg/L. MilliQ water was used as a blank sample. 0.2ml samples of each concentration were added to the kit test tubes containing TBPE and dichloromethane. The tubes were gently mixed for 2 minutes and allowed to stand for 30 minutes. They were then measured in a Lange DR2800 spectrophotometer in at 605nm in accordance with the Testing Kit instructions. Figure 2 shows the resulting calibration graph.

The filtrates were then diluted 1/10 with MilliQ water. 0.2 ml samples were measured in the spectrometer as before, and the concentration of the antifoam in each filtrate was read off from the calibration graph. The amount (%) of antifoam remaining in the filtrate was calculated as

$$\text{(measured concentration in filtrate)} / \text{(known starting concentration)} \times 100\%.$$

Antifoam concentrations down to 0.2mg/L ($2 \times 10^{-5}$ % w/v) can be measured by a similar technique, using the Lange LCK 333 Water Testing Kit, and constructing a calibration curve in the appropriate concentration range. In this case a 2ml aliquot of the sample to be measured is added to the test kit, rather than 0.2ml.

Fermentation process and removal of the antifoam

The fermentation to produce the foaming agent is carried out by culturing the host cell in a liquid fermentation medium within a bioreactor (e.g. an industrial fermenter). The composition of the medium (e.g. nutrients, carbon source etc.), temperature and pH are chosen to provide appropriate conditions for growth of the culture and/or production of the foaming agent. Air or oxygen-enriched air is normally sparged into the medium to provide oxygen for respiration of the culture.

The antifoam may be included in the initial medium composition and/or added as required through the period of the fermentation. Common practice is to employ a foam detection method, such as a conductivity probe, which automatically triggers addition of the antifoam. In the present invention, the antifoam is preferably present at a final concentration of from 0.1 to 20g/L, more preferably from 1 to 10g/L.

The fermenter temperature during step i), i.e. during fermentation, may be above or below the cloud point of the antifoam. Preferably the fermenter temperature is above the cloud point of the antifoam, since the antifoam is most effective at causing bubble coalescence and foam collapse above its cloud point. The fermenter temperature is generally chosen to achieve optimum conditions for growth of the host cells and / or production.

At the end of the fermentation, the antifoam must be substantially removed to ensure that the functionality of the foaming agent is not impaired. Removal of the antifoam is achieved by ensuring that the temperature of the fermentation medium is above the cloud point of the antifoam, so that the antifoam phase separates. The phase separated antifoam can be removed from the fermentation medium by any suitable method such as:

- filtration, e.g. dead-end filtration or a filter press
- membrane (cross-flow) filtration, e.g. microfiltration or ultrafiltration
- centrifugation
- adsorption, using e.g. activated carbon, silica or diatomaceous earth as an absorbent.

More antifoam is removed if the temperature of the fermentation medium is at least 10°C above the cloud point, preferably at least 20°C above the cloud point, most preferably at least 30°C above the cloud point. Preferably the temperature of the fermentation medium is less than 90°C, more preferably less than 75°C. In a preferred embodiment, the antifoam has a cloud point in the range 20-30°C and the temperature of the fermentation medium is in the range 40-60°C

A preferred method for separating the antifoam is membrane filtration. It has been generally thought that carrying out membrane filtration of fermentation broths containing an antifoam at temperatures above its cloud point results in fouling of the membrane by the precipitated antifoam, causing a low permeate flux and consequent processing difficulties: see for example Yamagiwa et al., J. Chem. Eng. Japan, 26 (1993) pp 13-18, and WO 01 / 014521. Thus it has previously been thought that membrane filtration should take place at temperatures below the cloud point. However, acceptable fluxes are obtained when carrying out ultrafiltration and microfiltration operations at a temperature of about 25°C above the cloud point of the antifoam.

In order to ensure that the product foaming agent is free from of intracellular and genetic material (which is usually regarded as an undesirable contaminant) the cells must be removed from the fermentation medium. In a preferred embodiment, the cells are separated from the medium at the same time as the precipitated antifoam is removed, for example in a microfiltration step which takes place at a temperature above the cloud point.

In an alternative embodiment the cells may be removed from the medium in a separate step prior to the removal of the antifoam - for example by filtration (e.g. dead-end filtration or a filter press), membrane / cross-flow filtration, (e.g. microfiltration or ultrafiltration), or centrifugation - at a temperature below the cloud point. In this embodiment, a purification and/or concentration step (e.g. by ultrafiltration) may be carried out (again at a temperature below the cloud point) after cell removal but before antifoam separation. The medium is then heated to a temperature above the cloud point so that the antifoam can be removed as already described.

Once the antifoam and the cells have been removed from the fermentation medium, the product foaming agent may be further purified and concentrated as required, e.g. by ultrafiltration. If the foaming agent is a hydrophopbin, it can be purified from the fermentation medium by, for example, the procedure described in WO01/57076 which involves adsorbing the hydrophobin to a surface and then contacting the surface with a surfactant, such as Tween 20, to elute the hydrophobin from the surface. See also Collen et al., 2002, Biochim Biophys Acta. 1569: 139-50; Calonje et al., 2002, Can. J. Microbiol. 48: 1030-4; Askolin et al., 2001, Appl Microbiol Biotechnol. 57: 124-30; and De Vries et al., 1999, Eur J Biochem. 262: 377-85.

The present invention will now be further described with reference to the following examples which are illustrative only and non-limiting.

Example 1: Removal of antifoam from a fermentation liquor containing a foaming agent

A fed-batch fermentation of a genetically modified strain of *Saccharomyces cerevisiae* was performed. The strain had been modified by incorporating the gene encoding the hydrophobin HFBII from the fungus *Trichoderma reesei* (a foaming agent) in such a way that extracellular expression of the hydrophobin was achieved during fermentation. Fermentation was carried out essentially as described by van de Laar T et al., in Biotechnol Bioeng. 96(3):483-94 (1997), using glucose as a carbon source and scaling the process to a total volume of 150L in a 300L fermentation vessel. The antifoam Struktol J647 was used to control foaming during the fermentation (instead of Struktol J673 used by van de Laar T *et al*).

At the end of the fermentation, the fermentation liquor was microfiltered at 15°C (i.e. below the cloud point of the antifoam J647) to remove the yeast cells. Microfiltration was performed on pilot scale plant with Kerasep ceramic membranes having a pore size of 0.1 μm, using two volumes of diafiltration with deionised water. The liquor was then ultrafiltered, again at 15°C, to partially purify the HFBII. Ultrafiltration was by 1kD Synder spiral wound polymeric membranes at a transmembrane pressure of 0.9 bar and four volumes of diafiltration.

The concentration of the antifoam in the fermentation liquor after the ultrafiltration step was measured to be 0.196g/L. The concentration of HFBII was measured to be 0.320g/L by high performance liquid chromatography (HPLC), as follows. The sample was diluted with 60% aqueous ethanol to give an approximate concentration of 200 μg/ml prior to analysis. HPLC separation was performed on a Vydac Protein C4 column (250 x 4.6 mm) at 30°C. Hydrophobin was measured by UV detection at 214nm and the concentration was calculated by comparison with samples of known HFBII concentration obtained from VTT Biotechnology (Espoo, Finland).

The cell-free liquor was then heated to 50°C, held at that temperature for 30 minutes, and filtered (0.2μm pore size) to remove the antifoam. The remaining amounts of antifoam and HFBII in the filtrate were measured as before and are given in Table 2 (Stage 1). The filtrate from this first stage was then re-heated to 50°C, held at this temperature for a further 30 minutes, and filtered as before. The HFBII and antifoam concentrations in the resulting filtrate were measured and are also given in Table 2 (Stage 2).

Table 2

|  | Stage 1 | Stage 2 |
|---|---|---|
| Amount of HFBII in filtrate (g/L) | 0.32 | 0.30 |
| % of initial HFBII concentration remaining | 100% | 93.75% |
| Amount of antifoam in filtrate (g/L) | 0.05 | .028 |
| % of initial antifoam concentration remaining | 25.5% | 14.3% |
| Mass ratio antifoam:hydrophobin | 0.156 | 0.093 |

The resulting hydrophobin solution was found to have satisfactory foaming properties.

The various features and embodiments of the present invention, referred to in individual sections above apply, as appropriate, to other sections, mutatis mutandis. Consequently features specified in one section may be combined with features specified in other sections, as appropriate.

All publications mentioned in the above specification are herein incorporated by reference. Various modifications and variations of the described methods of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in the relevant fields are intended to be within the scope of the following claims.

**Claims**

1. Aqueous solution containing at least 300 mg/l of hydrophobin and at least 0.3 mg/l of a antifoam with a cloud point, wherein the antifoam/hydrophobin weight ratio is below 0.2, preferably below 0.15, more preferably below 0.1.

2. Solution according to claim 1 wherein the hydrophobin is a class II hydrophobin,

3. Solution according to claim 2 wherein the hydrophobin is HFBI or HFBII from *Trichoderma reesei.*

4. Solution according to any preceding claims containing at least 1g/l, preferably at least 10g/l even more preferably at least 100g/l of hydrophobin.

**Patentansprüche**

1. Wässrige Lösung, enthaltend mindestens 300 mg/Liter Hydrophobin und mindestens 0,3 mg/Liter eines Antischaummittels mit einem Cloudpoint, wobei das Gewichtsverhältnis von Antischaummittel zu Hydrophobin unter 0,2, vorzugsweise unter 0,15 und insbesondere unter 0,1 liegt.

2. Lösung nach Anspruch 1, wobei es sich beim Hydrophobin um Hydrophobin der Klase II handelt.

3. Lösung nach Anspruch 2, wobei es sich beim Hydrophobin um HFB I oder HFB II aus Trichoderma reesei handelt.

4. Lösung nach einem der vorstehenden Ansprüche, enthaltend mindestens 1 g/Liter, vorzugsweise mindestens 10 g/Liter und insbesondere mindestens 100 g/Liter Hydrophobin.

**Revendications**

1. Solution aqueuse contenant au moins 300 mg/l d'hydrophobine et au moins 0,3 mg/l d'un antimousse avec un point de trouble, dans laquelle le rapport massique antimousse/hydrophobine est inférieur à 0,2, de préférence inférieur à 0,15, encore mieux inférieur à 0,1.

2. Solution selon la revendication 1, dans laquelle l'hydrophobine est une hydrophobine de classe II.

3. Solution selon la revendication 2, dans laquelle l'hydrophobine est HFBI ou HFBII de *Trichoderma reesei.*

4. Solution selon l'une quelconque des revendications précédentes contenant au moins 1 g/l, de préférence au moins 10 g/l bien mieux encore au moins 100 g/l d'hydrophobine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1623631 A
- WO 0174864 A
- WO 9641882 A
- WO 01014521 A
- WO 0157076 A

### Non-patent literature cited in the description

- **BAILEY et al.** *Appl. Microbiol. Biotechnol.,* 2002, vol. 58, 721-727
- **SAMBROOK et al.** Molecular Cloning: A Laboratory Manual. Cold Spring Harbor Laboratory Press, 2001
- Current Protocols in Molecular Biology. **AUSUBEL et al.** Short Protocols in Molecular Biology. John Wiley & Sons, Inc, 1999
- **MACCABE ; VAN ALFEN.** *App. Environ. Microbiol,* 1999, vol. 65, 5431-5435
- **DESAI ; BANAT.** *Microbiology and Molecular Biology Reviews,* March 1997, 47-64
- **WESSELS.** *Adv. Microb. Physio.,* 1997, vol. 38, 1-45
- **WOSTEN.** *Annu Rev. Microbiol.,* 2001, vol. 55, 625-646
- **DE VOCHT et al.** *Biophys. J.,* 1998, vol. 74, 2059-68
- **WOSTEN et al.** *Embo. J.,* 1994, vol. 13, 5848-54
- **TALBOT.** *Curr. Biol,* 2003, vol. 13, R696-R698
- **BETH JUNKER.** Foam and its mitigation in fermentation systems. *Biotechnology Progress,* 2007, vol. 23, 768-784
- Surfactant Aggregation and Adsorption at Interfaces. Colloid Science: Principles, Methods and Applications. Blackwell Publishing, 2005, 63
- **YAMAGIWA et al.** *J. Chem. Eng. Japan,* 1993, vol. 26, 13-18
- **COLLEN et al.** *Biochim Biophys Acta,* 2002, vol. 1569, 139-50
- **CALONJE et al.** *Can. J. Microbiol.,* 2002, vol. 48, 1030-4
- **ASKOLIN et al.** *Appl Microbiol Biotechnol.,* 2001, vol. 57, 124-30
- **DE VRIES et al.** *Eur J Biochem.,* 1999, vol. 262, 377-85
- **VAN DE LAAR T et al.** *Biotechnol Bioeng.,* 1997, vol. 96 (3), 483-94